# EUROPÄISCHE PATENTSCHRIFT

(11) **EP 0 819 678 B1**
(45) Veröffentlichungstag und Bekanntmachung des Hinweises auf die Patenterteilung: **18.02.2004**
(21) Anmeldenummer: 97111255.2
(22) Anmeldetag: 04.07.1997
(51) Int. Cl.: C07D 231/16

(54) **Neue Nitropyrazolester, ein Verfahren zu ihrer Herstellung und ihre Verwendung zur Herstellung eines Nitropyrazolamids**
New nitropyrazole-esters, a process for their preparation and their use for the preparation of a nitropyrazole-amide
Nouveaux esters de nitropyrazole, un procédé pour leur préparation et leur utilisation pour la préparation d'une amide de nitropyrazole

(30) Priorität: 17.07.1996 DE 19628778; 16.08.1996 DE 19632920
(43) Veröffentlichungstag der Anmeldung: 21.01.1998
(73) Patentinhaber: Bayer Chemicals AG, 51368 Leverkusen (DE)
(72) Erfinder: Heuer, Lutz, Dr., 41542 Dormagen (DE); Müller, Nikolaus, Dr., 40789 Monheim (DE); Steffan, Guido, Dr., 51519 Odenthal (DE)

(56) Entgegenhaltungen:
- EP-A- 0 463 756
- EP-A- 0 526 004
- CHEMICAL ABSTRACTS, vol. 52, no. 1, 10.Januar 1958 Columbus, Ohio, US; ROBINS ET AL.: "Potential purine and antagonists..." Spalte 395; XP002047057
- ELLAMES G J ET AL: "The syntheses of acycloformycins and 5-amino-3-(2-hydroxyethoxy)met hylpyrazolo[4,3-d]pyrimidin-7(6H)-one, an analog of the antiviral acycloguanosine" J. CHEM. SOC., PERKIN TRANS. 1 (JCPRB4,0300922X);85; (10); PP.2087-91, SEARLE RES. DEV.;DEP. MED. CHEM.; HIGH WYCOMBE; UK (GB), XP002047056

## Beschreibung

Die vorliegende Erfindung betrifft neue Nitropyrazolester, ein Verfahren zu ihrer Herstellung durch eine Nitrierung von entsprechenden Pyrazolestern und ihre Verwendung als Zwischenprodukte zur Herstellung eines bekanten Nitropyrazolamids, bei dem es sich seinerseits um ein Zwischenprodukt zur Herstellung von Pharmawirkstoffen handelt.

Es ist bekannt, daß man sich von den erfindungsgemäßen Nitropyrazolestern unterscheidende alkylsubstituierte Nitropyrazolester durch Veresterung der entsprechenden Säuren oder durch Alkylierung nicht-alkylierter Nitropyrazolester herstellen kann (siehe DE-A 30 29 281, Gazz. Chim. Ital. 75, 121 bis 130 (1945), J. Med. Chem. 37, 4329 bis 4337 (1994) und Acta Biochem. Pol. 27, 35 bis 56 (1980)).

Nachteilig ist bei diesen Verfahren, daß sie erst durch einen Umweg zum Ziel führen, wobei zunächst Pyrazolcarbonsäure hergestellt und isoliert, dann verestert und dann nitriert oder in umgekehrter Reihenfolge verfahren werden muß.

Weiterhin ist bekannt, daß man das Nitropyrazolamid der Formel (I) 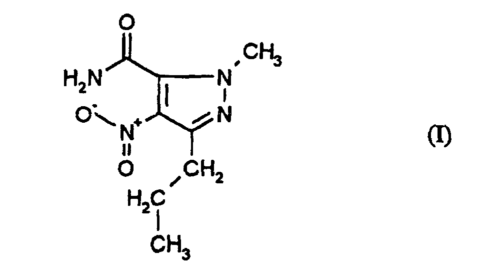 aus der entsprechenden Pyrazolcarbonsäure durch Nitrierung, Bildung des Säurechlorids und Umsetzung mit Ammoniak herstellen kann (siehe EP-A 0 463 756). Die Pyrazolcarbonssäure ihrerseits wird durch Verseifung eines intermediär auftretenden entsprechenden Esters erhalten.

Auch dieses Verfahren ist vielstufig und umständlich.

Es wurden nun Nitropyrazolester der Formel (II) gefunden, 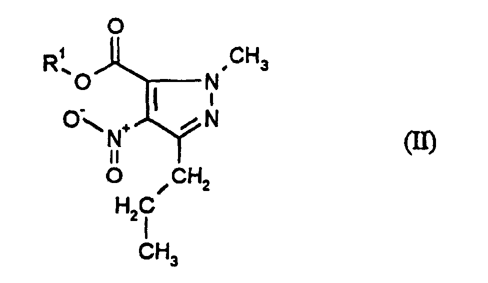 in der
- R¹: für C₁-C₆-Alkyl steht.

Vorzugsweise steht in Formel (II) R¹ für Methyl, Ethyl, n-Propyl, i-Propyl, n-Butyl, i-Butyl oder sek.-Butyl.

Die vorliegende Erfindung betrifft auch Mischungen von zwei oder mehr Nitropyrazolestern der Formel (II), in denen die einzelnen Komponenten in beliebigen Verhältnissen zueinander vorliegen können.

Die vorliegende Erfindung betrifft weiterhin ein Verfahren zur Herstellung von Nitropyrazolestern der Formel (II), das dadurch gekennzeichnet ist, daß man Pyrazolester der Formel 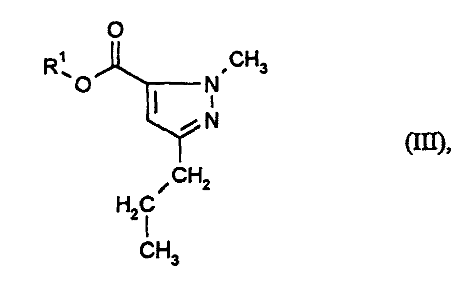 in der
- R¹: die bei Formel (II) angegebene Bedeutung hat, nitriert.

Pyrazolester der Formel (III) sind bekannt und auf bekannte Weise oder analog dazu zugänglich (siehe z. B. EP-A 463 756, C.A. 123, 198 791 (1995), C.A. 114, 185 497 (1991), EP-A 526 004 und CAS-Nr. 133 261-07-1).

Die Nitrierung kann beispielsweise mit einer nitrierend wirkenden Mischung aus Salpetersäure und Schwefelsäure, nur mit Salpetersäure oder durch Einbringen eines anorganischen Nitrats in Schwefelsäure durchgeführt werden. Beim Einsatz von Gemischen von Salpeter- und Schwefelsäure können diese z.B. im Gewichtsverhältnis von Salpetersäure zu Schwefelsäure von 99,9:0,1 bis 1:99, vorzugsweise 90:10 bis 10:90 vorliegen.

Das Nitrieragens kann man z. B. in der stöchiometrisch erforderlichen Menge oder im Überschuß einsetzen, beispielsweise bis zu 50-fachem Überschuß. Bevorzugt wird das 1,1- bis 10-fache der stöchiometrisch erforderlichen Menge eingesetzt.

Es ist vorteilhaft, Nitrieragenzien mit möglichst geringem Wassergehalt einzusetzen. Der Wassergehalt liegt z. B. unter 10 Gew.-%, bevorzugt zwischen 0 und 5 Gew.-%.

Die Nitrierung kann beispielsweise bei Temperaturen im Bereich -30 bis +100°C durchgeführt werden. Vorzugsweise arbeitet man bei -10 bis +70°C, besonders bevorzugt bei 0 bis +60°C.

Die Reaktionszeit ist von der Temperatur in der Weise abhängig, daß bei höheren Temperaturen kürzere Reaktionszeiten gewählt werden können. Sie kann beispielsweise zwischen 20 Minuten und 24 Stunden betragen. Bevorzugt sind Reaktionszeiten zwischen 1 und 10 Stunden.

Die Nitrierung kann mit oder ohne den Zusatz eines Lösungsmittels durchgeführt werden. Aus Gründen der besseren Handhabbarkeit des Reaktionsgemisches ist der Zusatz eines Lösungsmittels bevorzugt. Geeignete Lösungsmittel sind solche, die bei den angewendeten Bedingungen nicht nitriert werden können. Beispiele sind Schwefelsäure, Oleum, o-Dichlorbenzol, Dinitrobenzole, Dichlormethan und Dichlorethan.

Wenn man den hergestellten Nitropyrazolester der Formel (II) isolieren möchte, so kann man beispielsweise das ausreagierte Nitriergemisch auf Eis und/oder Wasser austragen, dann mit einem mit Wasser nicht mischbaren Lösungsmittel extrahieren und das Extrakt vom Lösungsmittel befreien.

Die vorliegende Erfindung betrifft auch die Verwendung von Nitropyrazolestern der Formel (II) zur Herstellung des Nitropyrazolamids der Formel (I), indem man Nitropyrazolester der Formel (II) mit Ammoniak umsetzt. Man kann diese Verwendung auch als ein Verfahren zur Herstellung des Nitropyrazolamids der Formel (I) bezeichnen, das dadurch gekennzeichnet ist, daß man Nitropyrazolester der Formel (II) mit Ammoniak umsetzt.

Die Umsetzung mit Ammoniak kann beispielsweise mit wäßrigem oder gasförmigem Ammoniak, bei beispielsweise 0 bis 250°C und beispielsweise in einem offenen Reaktor oder in einem geschlossenen Autoklaven unter Drucken bis beispielsweise 50 bar erfolgen. Bevorzugte Reaktionsbedingungen sind Temperaturen im Bereich 20 bis 120°C, insbesondere 30 bis 100°C und Drucke von Normaldruck bis zu dem sich bei der jeweiligen Reaktionstemperatur von selbst einstellenden Druck des Reaktionssystems in einem Autoklaven.

Ammoniak kann beispielsweise in Mengen zwischen der stöchiometrisch erforderlichen Menge und dem 50-fachen Überschuß davon eingesetzt werden. Bevorzugt ist die 1,2- bis 20-fache Menge der stöchiometrisch erforderlichen Menge.

Die Umsetzung mit Ammoniak kann mit oder ohne den Zusatz eines Lösungsmittels durchgeführt werden. Vorzugsweise arbeitet man unter Zusatz eines inerten organischen Lösungsmittels. Beispiele hierfür sind: Alkohole wie Methanol, Ethanol, Propanol und Butanole, Aromaten wie Benzol, Toluol, Chlorbenzol und Dichlorbenzole, und Sulfoxide wie Dimethylsulfoxid. Es können auch Lösungsmittelgemische eingesetzt werden.

Als Katalysatoren für die Umsetzung mit Ammoniak können gegebenenfalls Basen z.B. in Mengen von 0,05 bis 5 mol-% eingesetzt werden. Geeignet sind z.B. Alkoholate wie Kalium-tert.-butylat, Natriumethylat, Natriummethylat, Natriumisopentanoat und Natriumisopropylat sowie andere Basen wie DABCO, Natriumhydroxid, Kaliumhydroxid, Natriumcarbonat, Kaliumcarbonat, DBU, DBM und Triethylamin.

Aus dem nach der Umsetzung mit Ammoniak vorliegenden Reaktionsgemisch kann man das hergestellte Nitropyrazolamid der Formel (I) z.B. isolieren, indem man die flüchtigen Komponenten des Reaktionsgemischs im Vakuum abzieht.

Bei einer besonderen Ausführungsform setzt man Pyrazolester der Formel (III) mit einer nur gerade ausreichenden Menge oder einem geringen Überschuß an Nitriermittel um und führt anschließend ohne Zwischenisolierung die Umsetzung mit Ammoniak durch.

Das erfindungsgemäß über die Zwischenstufe von Nitropyrazolestern der Formel (II) hergestellte Nitropyrazolamid der Formel (I) kann wie auf andere Weise erhaltenes Nitropyrazolamid der Formel (I) verwendet werden, beispielsweise zur Herstellung von Wirkstoffen (siehe z. B. EP-A 463 756).

Die neuen Nitropyrazolester der Formel (II) können einstufig aus entsprechenden Pyrazolestern der Formel (III) erhalten werden und machen das Nitropyrazolamid der Formel (I) ebenfalls einstufig zugänglich. Der Syntheseweg zum Nitropyrazolamid der Formel (I) wird gegenüber dem Stand der Technik verkürzt, die erzielbaren Raum-Zeit-Ausbeuten sind verbessert und es werden geringere Mengen an Reagenzien und Energie als bisher benötigt. Es ist überraschend, daß diese vorteilhaften Effekte mit dem wenig aufwendigen erfindungsgemäßen Verfahren realisierbar sind, denn die Fachwelt hat bisher nur kompliziertere Verfahren zur Herstellung des Nitropyrazolamids der Formel (I) in Betracht gezogen.

### Beispiele

### Beispiel 1

Bei 45°C wurden zu einer Lösung aus 86,1 g 89 gew.-%igem 1-Methyl-3-propyl-5-pyrazolcarbonsäureethylester (A) in 330 ml konzentrierter Schwefelsäure 193,7 g einer Mischung aus 33,3 Gew.-% konzentrierter Salpetersäure und 66,7 Gew.-% konzentrierter Schwefelsäure langsam hinzugetropft. Das Gemisch wurde 3 weitere Stunden nachgerührt, danach auf Raumtemperatur abgekühlt und auf 2 kg Eiswasser gegeben. Das dann vorliegende Gemisch wurde mit Toluol extrahiert. Nach dem Trocknen der Toluolphase bei 50°C im Vakuum wurden 102 g eines hellgelben Öls erhalten, bei dem es sich um 1-Methyl-3-propyl-4-nitro-5-carbonsäureethylester (B) handelte. Das entspricht einer Ausbeute von 97 % derTheorie.

### Beispiel 2

Bei 50°C wurden zu einer Lösung von 120 g 89 gew.-%igem 1-Methyl-3-propyl-4-nitro-5-pyrazolcarbonsäureethylester in 400 ml Ethanol in einem Autoklaven 85 g gasförmiges Ammoniak aufgepreßt. Das Reaktionsgemisch wurde noch 3 Stunden nachgerührt und dann durch Abziehen des Lösungsmittels 103 g 1-Methyl-3-propyl-4-nitro-5-carbonsäureamid (C) als farblose Nadel mit einem Schmelzpunkt von 140°C erhalten.

### Beispiel 3

In einer Stunde wurden 50 g (A) und 92,5 g Schwefelsäure zu einem Gemisch aus 62 g Schwefelsäure, 33,7 g 20gew.-%igem Oleum und 65 g Salpetersäure getropft. Es wurde 1 Stunde bei 45°C nachgerührt und dann wie in Beispiel 1 beschrieben aufgearbeitet. Es wurden 52,2 g (B) erhalten.

### Beispiel 4

50 g (B), 14,5 g Ammoniak und 110 g Ethanol wurden 20 Stunden bei 50°C und wie in Beispiel 2 beschrieben, gerührt und aufgearbeitet. Es wurden 37,3 g (C) erhalten.

## Patentansprüche

1. Nitropyrazolester der Formel 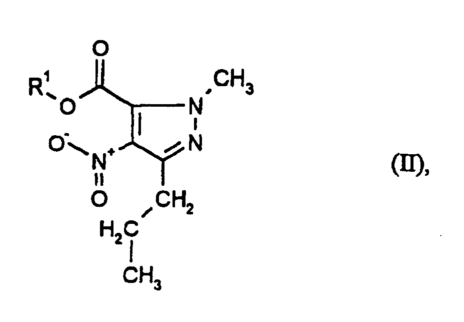 in der
R¹ für C₁-C₆-Alkyl steht.

2. Nitropyrazolester nach Anspruch 1, **dadurch gekennzeichnet, daß** es sich um Mischungen von zwei oder mehr Nitropyrazolestern der Formel (II) handelt.

3. Verfahren zur Herstellung von Nitropyrazolestern des Anspruchs 1, **dadurch gekennzeichnet, daß** man Pyrazolester der Formel 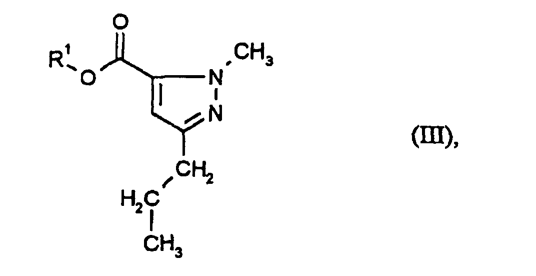 in der
R¹ die im Anspruch 1 bei Formel (II) angegebene Bedeutung hat, nitriert.

4. Verfahren nach Anspruch 3, **dadurch gekennzeichnet, daß** man die Nitrierung mit einer nitrierend wirkenden Mischung aus Salpetersäure und Schwefelsäure, nur mit Salpetersäure oder durch Einbringen eines anorganischen Nitrats in Schwefelsäure durchführt.

5. Verfahren nach Ansprüchen 3 und 4, **dadurch gekennzeichnet, daß** man das Nitrieragens in einer Menge zwischen der stöchiometrisch erforderlichen Menge und einem 50-fachen Überschuß davon einsetzt.

6. Verfahren nach Ansprüchen 3 bis 5, **dadurch gekennzeichnet, daß** man die Nitrierung bei Temperaturen im Bereich -30 bis +100°C und unter Zusatz eines Lösungsmittels durchführt.

7. Verwendung von Nitropyrazolestern des Anspruchs 1 zur Herstellung des Nitropyrazolamids der Formel 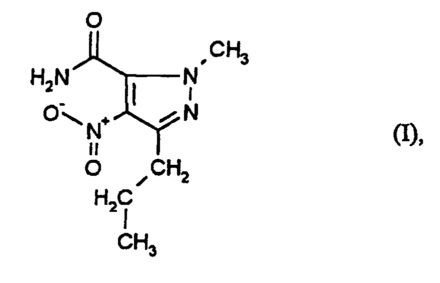 indem man Nitropyrazolester der Formel (II) mit Ammoniak umsetzt.

8. Verfahren zur Herstellung des Nitropyrazolamids der Formel 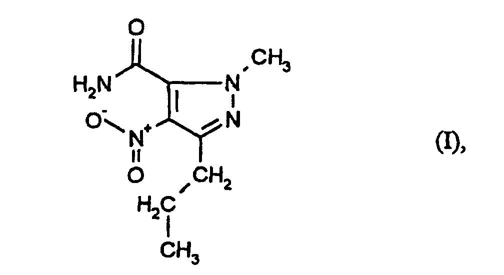 das **dadurch gekennzeichnet ist, daß** man Nitropyrazolester des Anspruchs 1 mit Ammoniak umsetzt.

9. Verfahren nach Anspruch 8, **dadurch gekennzeichnet, daß** man die Umsetzung mit Ammoniak mit wäßrigem oder gasförmigem Ammoniak bei 0 bis 250°C und Drucken bis 50 bar durchführt.

10. Verfahren nach Ansprüchen 8 und 9, **dadurch gekennzeichnet, daß** man Ammoniak in Mengen von der stöchiometrisch erforderlichen Menge bis zu dem 50-fachen Überschuß davon einsetzt und ein Lösungsmittel zusetzt.

## Claims

1. Nitropyrazole ester of the formula 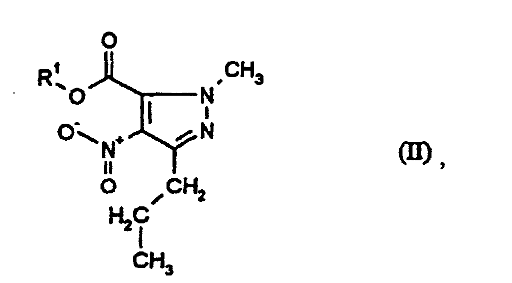 in which
R¹ represents C₁-C₆-alkyl.

2. Nitropyrazole ester according to Claim 1, **characterized in that** it is a mixture of two or more nitropyrazole esters of the formula (II).

3. Process for the preparation of nitropyrazole esters of Claim 1, **characterized in that** it comprises nitrating pyrazole esters of the formula 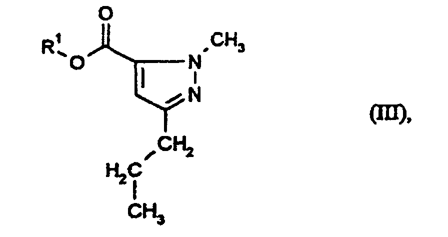 in which
R¹ has the meaning indicated in Claim 1 under formula (II).

4. Process according to Claim 3, **characterized in that** the nitration is carried out using a mixture, having a nitrating action, of nitric acid and sulphuric acid only using nitric acid or by introduction of an inorganic nitrate into sulphuric acid.

5. Process according to Claims 3 and 4, **characterized in that** the nitrating agent is employed in an amount between the stoichiometrically required amount and a 50-fold excess thereof.

6. Process according to Claims 3 to 5, **characterized in that** the nitration is carried out at temperatures in the range -30 to +100°C and with addition of a solvent.

7. Use of nitropyrazole esters of Claim 1 for the preparation of the nitropyrazole amide of the formula 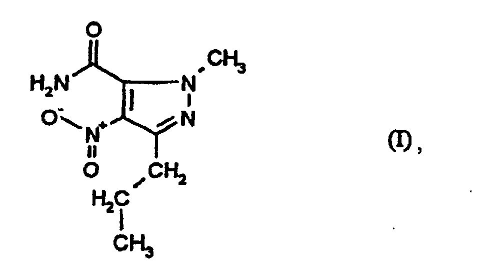 by reacting nitropyrazole esters of the formula (II) with ammonia.

8. Process for the preparation of the nitropyrazole amide of the formula 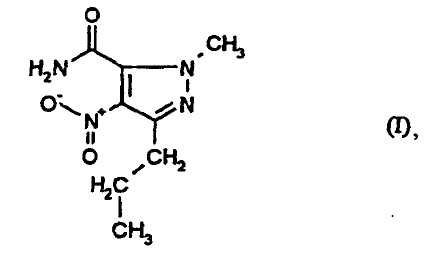 which is **characterized in that** it comprises reacting nitropyrazole esters of Claim 1 with ammonia.

9. Process according to Claim 8, **characterized in that** the reaction with ammonia is carried out using aqueous or gaseous ammonia at 0 to 250°C and pressures up to 50 bar.

10. Process according to Claims 8 and 9, **characterized in that** ammonia is employed in amounts from the stoichiometrically required amount up to a 50-fold excess thereof and a solvent is added.

## Revendications

1. Esters de nitropyrazole de formule 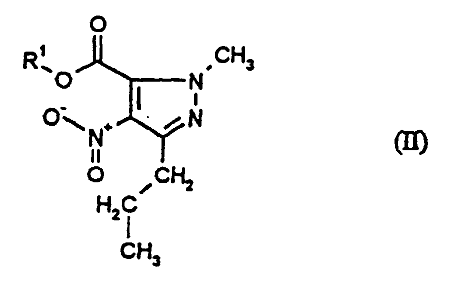 dans laquelle
R¹ désigne un radical alkyle C₁-C₆.

2. Esters de nitropyrazole selon la revendication 1, **caractérisés en ce qu'**il s'agit de mélanges de deux esters de nitropyrazole ou plus de formule (11).

3. Procédé de préparation d'esters de nitropyrazole selon la revendication 1, **caractérisé en ce que** des esters de pyrazole de formule
dans laquelle 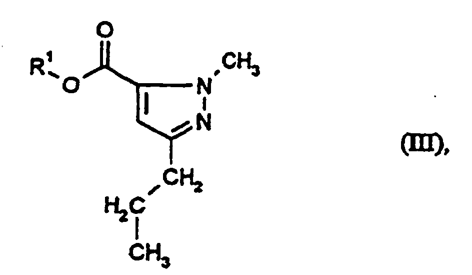
R¹ a la signification spécifiée dans la revendication 1 pour la
formule (II)
sont nitrés.

4. Procédé selon la revendication 3, **caractérisé** en ce la nitration est effectuée avec un mélange nitrant d'acide nitrique et d'acide sulfurique, uniquement avec de l'acide nitrique ou par introduction d'un nitrate inorganique dans de l'acide sulfurique.

5. Procédé selon les revendications 3 et 4, **caractérisé en ce que** l'agent de nitration est utilisé dans une quantité entre la quantité stoechiométrique nécessaire et un excédent de 50 fois.

6. Procédé selon l'une des revendications 3 à 5, **caractérisé en ce que** la nitration est effectuée à des températures de l'ordre de -30 à +100°C et moyennant addition d'un solvant.

7. Mise en oeuvre d'esters de nitropyrazole de la revendication 1 pour la préparation du nitropyrazolamide de formule 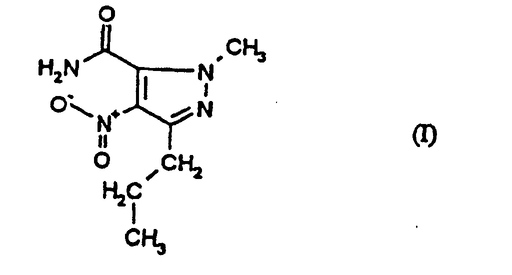 en faisant réagir des esters de nitropyrazole de formule (II) avec de l'ammoniac.

8. Procédé de préparation de nitropyrazolamide de formule 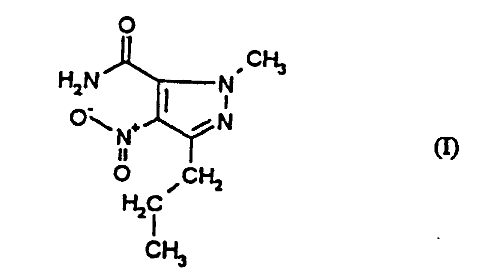 qui est **caractérisé en ce que** des esters de nitropyrazole de la revendication 1 sont mis en réaction avec de l'ammoniac.

9. Procédé selon la revendication 8, **caractérisé en ce que** la mise en réaction avec de l'ammoniac est effectuée avec de l'ammoniaque aqueux ou de l'ammoniac gazeux à une température de 0 à 250°C et une pression jusqu'à 50 bars.

10. Procédé selon l'une des revendications 8 et 9, **caractérisé en ce que** l'ammoniac est utilisé dans des quantités allant de la quantité stoechiométrique nécessaire à un excédent de 50 fois et qu'un solvant est ajouté.
